# EUROPEAN PATENT SPECIFICATION

(11) **EP 4 027 961 B1**
(45) Date of publication and mention of the grant of the patent: **21.06.2023**
(21) Application number: 20772428.7
(22) Date of filing: 09.09.2020
(51) Int. Cl.: A61F 13/00, A61F 13/02, A61M 1/00

(54) **SUPERABSORBENT DRESSING WITH FELTED FOAM LAYERS**
SUPERABSORBIERENDER WUNDVERBAND MIT GEFILZTEN SCHAUMSTOFFSCHICHTEN
PANSEMENT SUPERABSORBANT À COUCHES DE MOUSSE FEUTRÉE

(30) Priority: 10.09.2019 US 201962898265 P
(43) Date of publication of application: 20.07.2022
(73) Proprietor: KCI Licensing, Inc., San Antonio, TX 78265 (US)
(72) Inventor: ROBINSON, Timothy Mark, San Antonio, Texas 78265 (US); LOCKE, Christopher Brian, San Antonio, Texas 78265 (US); LONG, Justin Alexander, San Antonio, Texas 78265 (US)
(74) Representative: Simmons & Simmons
(86) International application number: PCT/IB2020/058367
(87) International publication number: WO 2021/048751

(56) References cited:
- EP-A1- 2 659 865
- WO-A1-2020/056014
- WO-A2-2020/003067
- US-A1- 2019 083 322

## Description

### BACKGROUND

The present disclosure relates generally to the field of wound therapy, and more particularly to absorbent dressings. Absorbent dressings are configured to absorb fluid exuded from a wound. Absorbent dressings can be improved by increasing flexibility and conformability of the dressing to a patient's anatomy, increasing fluid capacity for the dressing, and reducing a risk of ingrowth of a wound to the dressing, among other possible types of improvement.

US 2019/083322 A1 concerns a negative pressure wound dressing. EP 2659865 A1 concerns a wound dressing.

Some embodiments of the present disclosure relate to absorbent dressings for use with negative pressure wound therapy. Negative pressure wound therapy (NPWT) involves applying negative pressure (relative to atmospheric pressure) to a wound bed to promote wound healing. Typically, a dressing is sealed over a wound bed and air is pumped out of the dressing to create a negative pressure at the wound bed. In some NPWT systems, air is pumped out of the dressing while the dressing is used to absorb fluid from the wound. In such systems, the dressing preferably provides for manifolding (e.g., airflow and communication of air pressure) across the dressing, even when the dressing absorbs fluid. Accordingly, absorbent dressings with improved manifolding capabilities are desirable.

### SUMMARY

One implementation of the present disclosure is a wound dressing according to claim 1.

In some embodiments, the first felted foam layer and the second felted foam layer include an open-cell foam. The open-cell foam is felted to approximately one-fifth of an original thickness.

In some embodiments, the superabsorbent material is printed on the first felted foam layer as a slurry comprising a solvent and the superabsorbent material. The superabsorbent material may at least partially interlock with the first felted foam layer. The superabsorbent may also be printed on the second felted foam layer. The superabsorbent material may be arranged as a pattern of discrete deposits on the first felted foam layer.

In some embodiments, the dressing includes a drape coupled to the second felted foam layer. The drape includes a polyurethane material having a high moisture vapor transfer rate and an adhesive border configured to be coupled to a periwound area. The dressing may also include a connection pad coupled to the drape. The connection pad is configured to facilitate pneumatic communication between the first and second felted foam layers and a pump configured to draw a negative pressure at the wound dressing.

In some embodiments, the first felted foam layer is welded to the second felted foam layer.

Another implementation of the present disclosure is a method of manufacturing a dressing. the method includes obtaining an open-cell foam, heating and compressing the open-cell foam to create a felted foam, printing a superabsorbent material onto a first surface of the felted foam, dividing the felted foam into a first section and a second section, and coupling the first section to the second section such that the first surface of the first section faces the first surface of the second section.

In some embodiments, printing the superabsorbent material onto the first surface of the felted foam includes dissolving the superabsorbent material in a solvent to form a slurry and depositing the slurry in a plurality of discrete deposits on the first surface of the felted foam. The slurry may at least partially penetrate the felted foam. The method may also include allow the solvent to evaporate. The superabsorbent material can become at least partially interlocked with the felted foam when the solvent evaporates.

In some embodiments, heating and compressing the open-cell foam to create the felted foam includes heating the open-cell foam to approximately 150 degrees Celsius, and compressing the open-cell foam to approximately one-fifth of an original thickness of the open-cell foam. The method may also include skiving the felted foam to a thickness in a range of approximately two millimeters to approximately eight millimeters.

In some embodiments, coupling the first section to the second section includes welding the first section to the second section along a perimeter of the first section and the second section. Welding the first section to the second section can include applying a radio-frequency electromagnetic filed to the first section and the second section.

In some embodiments, the method includes coupling a polyurethane drape to the first section and providing the polyurethane drape with an adhesive configured to adhere the polyurethane drape to a periwound area. The method may also include coupling a connection pad to the polyurethane drape, coupling a tube to the connection pad, and coupling a pump to the tube. The pump is operable to draw air out of the felted foam via the connection pad and the tube.

### BRIEF DESCRIPTION OF THE DRAWINGS

FIG. 1 is a block diagram of a negative pressure wound therapy (NPWT) system with an absorbent dressing, according to an exemplary embodiment.
FIG. 2 is a top view of a portion of the NPWT system of FIG. 1, according to an exemplary embodiment.
FIG. 3 is a flowchart of a process of manufacturing the absorbent dressing of FIGS. 1-2, according to an exemplary embodiment.

### DETAILED DESCRIPTION

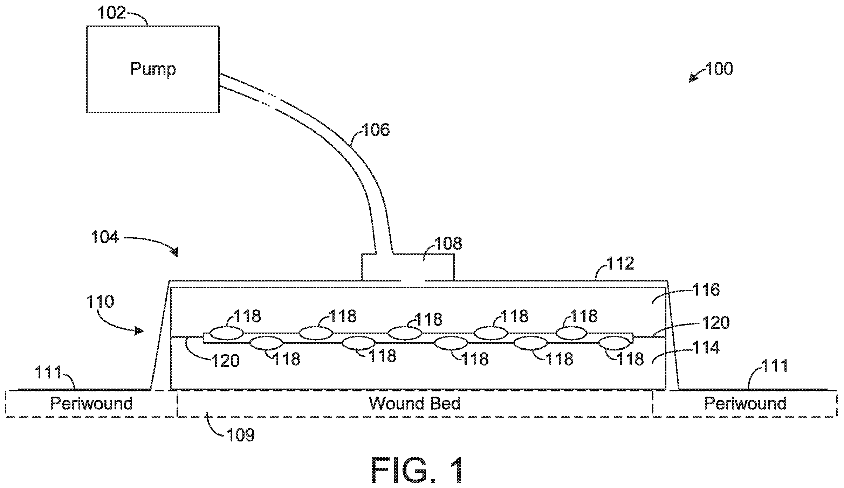

Referring now to FIGS. 1-2, a negative pressure wound therapy (NPWT) system 100 with a dressing 104 is shown, according to an exemplary embodiment. The NPWT system 100 includes a pump 102 pneumatically communicable with a dressing 104 via tube 106 (i.e., such that air can be transferred between the pump 102 and the dressing 104). In other embodiments, the pump 102 is fluidly communicable with the dressing 104 via the tube 106 (i.e., such that liquid, wound exudate, etc. in addition to air can be moved out of the dressing 104 via the tube 106). The tube 106 is coupled to the dressing 104 by a connection pad 108. The dressing 104 is shown as sealed over a wound bed 109. The wound bed 109 is a tissue wound of a patient, for example a laceration, burn, sore, trauma wound, chronic wound, etc.

Although the dressing 104 is described below in use with NPWT, the dressing 104 and/or variations thereof may also be suitable for use as an advanced wound dressing without the application of negative pressure.

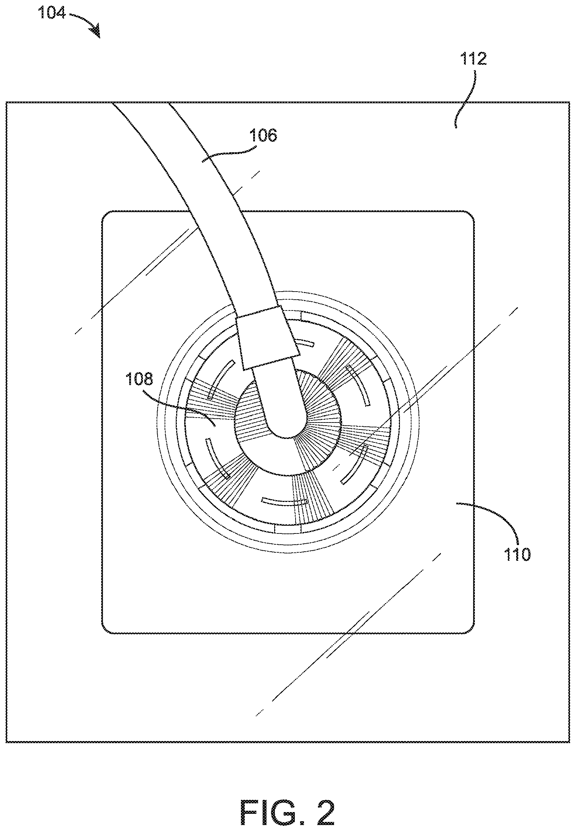

The dressing 104 is shown in a cross-sectional view in FIG. 1 and from atop view in FIG. 2. The dressing 104 allows a negative pressure to be maintained at the wound bed 109 while absorbing fluid from the wound bed 109. The dressing 104 thereby provides both negative pressure manifolding and a high level of fluid absorption. The dressing 104 is shown to include a drape 112 and a "tea-bag" structure 110 (i.e., top and bottom porous layers having an absorbent material disposed within).

The drape 112 is configured to seal the tea-bag structure 110 over the wound bed 109. For example, the drape 112 may include an adhesive border 111 coupleable to the patient's skin surrounding the wound bed 109. The drape 112 may include a material that substantially prevents leaking of air therethrough to facilitate creation and maintenance of a negative pressure at the tea-bag structure 110 (i.e., in a volume between the drape 112 and the wound bed 109). The drape may be a hydrophilic polymer film (e.g., polyurethanes) with a thickness in a range between approximately 15 microns and approximately 50 microns. The drape 112 may also include a material with a high moisture vapor transfer rate to facilitate evaporation of fluid to the ambient air through the drape 112, for example having a MVTR (upright cup) of greater than approximately 2600g/m²/day for a 30 micron film. The adhesive border 111 may include a silicone adhesive and/or an acrylic adhesive and is configured to provide a substantially air-tight seal between periwound area (e.g., skin surrounding the wound bed 109) and the drape 112. The adhesive border 111 may be configured to seal the dressing 104 over the wound bed 109 for a period of approximately seven days, in some embodiments.

The connection pad 108 is configured to couple the dressing 104 to a tube 106, which is coupled to a pump 102. The connection pad 108 is positioned at an airway (e.g., hole) extending through the drape 112 such that the connection pad 108 is in fluid communication with the tea-bag structure 110. The connection pad 108 is configured to allow airflow between the tea-bag structure 110 and the pump 102. In some embodiments, the connection pad 108 restricts (e.g., at least partially prevents) the flow of fluid from the dressing 104 to the pump 102, for example to reduce a risk of damage to the pump 102 or contamination of the pump 102. The tube 106 is configured to provide an airway that allows air to flow from the connection pad 108 to the pump 102.

The pump 102 is operable to pump air out of the dressing 104 (e.g., out of a volume between the drape 112 and the wound bed 109) via the tube 106 to create and maintain a negative pressure at the wound bed 109. In some embodiments, the pump 102 is electrically powered and the NPWT system 100 includes power systems and control circuitry to power and control operation of the pump 102. For example, the NPWT system 100 may include one or more pressure sensors or various other sensors that collect data used to control the pump 102 to maintain a negative pressure at the wound bed 109. In some embodiments, the pump 102 is manually-powered, such that a user may manipulate the pump 102 to draw air out of the dressing 104 as desired by the user. For example, the pump 102 may be spring-loaded to gradually pull air from the dressing 104 for a duration of time following a compression of the pump 102 by the user. The negative pressure applied at the wound bed may be in a range between approximately 75 mmHg and 150 mmHG, for example approximately 125 mmHg, and may be applied constantly, cyclically, or according to some other pattern.

The NPWT system 100 is thereby configured to provide a negative pressure at the wound bed 109 while also facilitating absorption of fluid from the wound bed 109 by the dressing 104. The NPWT system 100 may be configured such that the dressing 104 can be applied to the wound bed 109 for up to seven days between dressing changes. That is, the dressing 104 is obtained, sealed over a wound bed using adhesive of the drape 112, and coupled to the pump 102 via the tube 106. The pump can then be operated as described above to apply negative pressure therapy to the wound bed. The dressing 104 may be removed and replaced after up to approximately seven days.

The tea-bag structure 110 includes a first felted foam layer 114, a second felted foam layer 116 coupled to the first felted foam layer 114, and a superabsorbent material positioned between the first felted foam layer 114 and the second felted foam layer 116. The tea-bag structure 110 is configured to provide manifolding (e.g., air flow, communication of air pressure) through the tea-bag structure 110 to allow a negative pressure to be established and maintained at the wound bed 109. The tea-bag structure 110 is also configured to provide for absorption of fluid exuded from the wound bed 109. The tea-bag structure 110 is configured to be flexible when dry and to remain flexible when fluid is absorbed by the tea-bag structure 110. The tea-bag structure 110 thereby facilitates conformability of the dressing 104 to a patient's anatomy (which may have an irregular, curved geometry) and prevents the dressing 104 from pulling or lifting away from the periwound and wound bed when swollen with absorbed fluid.

The first felted foam layer 114 and the second felted foam layer 116 include a felted open-cell foam. The open-cell foam may be a polymeric, reticulated foam. The open-cell foam may be a polyether or polyester or mix-based polyurethane, classed as hydrophobic, with a pore size of approximately 17.7 pores per cm (45 pores per inch). The open-cell foam is heated and compressed (i.e., felted) to approximately one-fifth of an original thickness (i.e., approximately 5-times felted) to create the material used in the first felted foam layer 114 and the second felted foam layer 116 in the embodiment. In other embodiments, various other degrees of felting may be used, for example in a range between approximately 2-times felted and approximately 10-times felted. The felted foam of the felted foam layers 114, 116 is denser than the original open-cell foam while preserving the open-cell structure such that air can flow through the felted foam. After felting, the pore size of the felted foam may be in a range between approximately 35.4 pores per cm (90 pores per inch) to about 141.7 pores per cm (360 pores per inch), depending on the degree of felting. The first felted foam layer 114 and the second felted foam layer 116 may each have a thickness in a range of approximately 2 millimeters to approximately 8 millimeters. In some embodiments, the open-cell foam which is felted to form the felted foam layers 114, 116 is an open-cell foam marketed as GRANUFOAM^{™} by ACELITY^{™}. The foam material may be suitable for direct contact with the wound bed 109. For example, the first felted foam layer 114 may substantially prevent ingrowth of the healing wound bed 109 to the dressing 104.

The tea-bag structure 110 includes a superabsorbent material printed onto the first felted foam layer 114 and/or the second felted foam layer 116 in a plurality of deposits 118. The deposits 118 may be arranged in various patterns (e.g., lines, arrays, geometric distributions, random distributions) on the first felted foam layer 114 and/or the second felted foam layer 116. The superabsorbent material may include a super absorbent polymer. For example, the superabsorbent material may include polyacrylic and methacrylic and copolymers and may be mixed or copolymerized with polyacrylamides and other polymers such as 2-acrylamido-2-methylporpanesulfonic acid, and N-methyl pyrrolidones. The superabsorbent material may be ground to a fine particulate and dispersed in a solvent (e.g., polyvinyl alcohol, isopropyl alcohol, water, some combination thereof) to form a slurry. In some embodiments, a carrier polymer is used (e.g., polyvidone). In some embodiments, the slurry is softened with plasticizers (e.g., glycerol or polyethylene glycols). The slurry can be printed (e.g., forced through a nozzle controllable to move in two or three dimensions) onto the first felted foam layer 114 and/or the second felted foam layer 116 in a desired pattern. The slurry may have a gel- or paste-like consistency and can at least partially penetrate (seep into, flow into, etc.) the felted foam.

The solvent may then evaporate, leaving the superabsorbent material deposited on a surface of the felted foam and at least partially interlocked (coupled, bound, mixed, retained) with the felted foam. This interlocking at least partially prevents migration (translation, movement, etc.) of the superabsorbent deposits 118 relative to the first felted foam layer 114 and/or the second felted foam layer 116.

As shown in FIG. 1, the superabsorbent deposits 118 are separated from one another, which allows the tea-bag structure 110 to flex (bend, conform, etc) in the spaces between the superabsorbent deposits 118. Accordingly, the superabsorbent deposits 118 may be arranged in a pattern that improves the flexibility of the dressing 104. For example, the superabsorbent deposits 118 may be positioned at the cross points of a mesh pattern, where the mesh has a square form and a spacing in a range between approximately 8 mm and 15 mm, for example 10 mm. The superabsorbent deposits 118 may thereby be arranged in an equally-spaced array pattern. Because the superabsorbent deposits 118 are at least partially prevented from migrating relative to the first felted foam layer 114 and/or the second felted foam layer 116, the pattern of the superabsorbent deposits 118 can be substantially preserved during transportation and use of the dressing 104. Thus, the flexibility of the dressing 104 is also preserved, thereby improving the conformability of the dressing 104 to a patient's anatomy and substantially preventing the dressing 104 from assuming a planar form and pulling away from the wound bed 109 when swollen with fluid (which may be a prevalent tendency in other absorbent dressings).

The superabsorbent deposits 118 may also be arranged in a pattern that allows airflow between and around the superabsorbent deposits 118 through the tea-bag structure 110, even when the superabsorbent deposits 118 swell in response to absorbing fluid. Accordingly, both fluid absorption and manifolding of air is provided across substantially the entire area of the tea-bag structure 110 (and substantially the entire surface area of the wound bed 109). The tea-bag structure 110 may therefore provide improved airflow dynamics as compared to other superabsorbent dressings in which airflow is blocked at a large region of the dressing by a mass of superabsorbent material.

As shown in FIG. 1 the first felted foam layer 114 is welded to the second felted foam layer 116 along a border of the tea-bag structure 110, at welds 120. That is, the first felted foam layer 114 and the second felted foam layer 116 may be partially melted and joined at the welds 120 to bind the first felted foam layer 114 to the second felted foam layer 116. In other embodiments an adhesive is used to join the felted foam layers 114, 116. The superabsorbent deposits 118 are substantially confined between the first felted foam layer 114 and the second felted foam layer 116 (i.e., within the tea-bag structure). The density and the size of the pores of the open-cell material of the first felted foam layer 114 and the second felted foam layer 116 may be sufficient to prevent the superabsorbent material from migrating through the foam material to escape the tea-bag structure 118, while allowing the flow of air and fluid therethrough. The superabsorbent material may thereby be advantageously prevented from contacting the wound bed 109 and entering the tube 106.

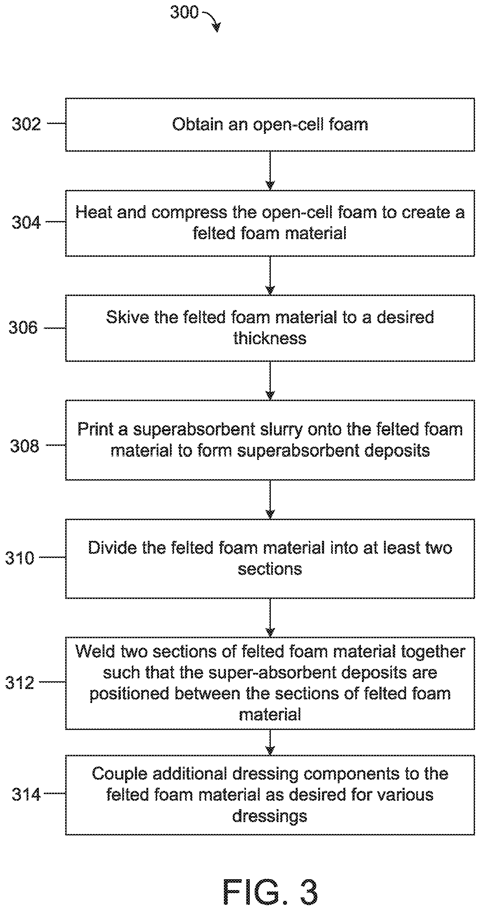

Referring now to FIG. 3, a flowchart of a process 300 for manufacturing an absorbent dressing (e.g., dressing 104) is shown, according to an exemplary embodiment. The process 300 may be used to manufacture the dressing 104 of FIGS. 1-2 and/or various other embodiments of absorbent dressings.

At step 302, an open-cell foam is obtained. The open-cell foam may be acquired from a supplier and/or manufactured at step 302. For example, to manufacture the open-cell foam, raw materials are mixed to trigger a chemical reaction. The mixture is placed in cast (mold) and allowed to rise to form the foam. The foam is removed from the cast. The open-cell foam obtained at step 302 may be an open-cell foam marketed as GRANUFOAM^{™} by ACELITY^{™} or other suitable open-cell foam. The open-cell foam may have a pore size of 17.7 pores per cm (45 pores per inch).

At step 304, the open-cell foam is heated and compressed to create a felted foam. For example, the open-cell foam may be heated to approximately 150 degrees Celsius and compressed to approximately one-fifth of the original thickness of the foam. In other embodiments, the open-cell foam is compressed to a thickness in a range of approximately one-half to one-eighth of the original thickness. The combination of heat and compression causes the open-cell foam to stay compressed (i.e., "felted") after the heat and compressive force are removed. The felted foam is thereby created, and may have a pore size in a range between 35.4 pores per cm (90 pores per inch) to 141.7 pores per cm (360 pores per inch), depending on the degree of compression at step 304.

At step 306, the felted foam is skived to a desired thickness. For example, the open-cell foam may be obtained with a thickness of approximately 100 millimeters, such that the felted foam has a thickness of approximately 20 millimeters. At step 306, the felted foam may then be cut down (skived) to a desired thickness in a range between approximately 2 millimeters and approximately 8 millimeters. In other embodiments, the open-cell foam is obtained with an initial thickness selected such that the felted material is already produced at the desired thickness and step 306 can be omitted.

At step 308, a superabsorbent slurry is printed onto the felted foam to form superabsorbent deposits on the felted foam. For example, a superabsorbent polymer maybe formed into a powder and dissolved in a solvent to form a slurry. The slurry may then be pumped or otherwise forced through a nozzle which is controllable in two, three, or more degrees of freedom. The slurry is selectively forced through the nozzle onto the felted foam to position the superabsorbent deposits in a pre-planned pattern. The slurry may be allowed to dry (i.e., provided time for the solvent to evaporate) to facilitate interlocking of the superabsorbent deposits and the felted foam.

At step 310, the felted foam is divided into at least two sections. For example, the felted foam may be cut into separate pieces to from the first felted foam layer 114 and the second felted foam layer 116 as shown in FIG. 1. The sections may be various shapes in various embodiments (e.g., rectangular, circular, oval-shaped, etc.). As another example, the felted foam is folded over onto itself (e.g., in half) to divide the felted foam into at least two sections.

At step 312, two sections of felted foam are welded together such that the superabsorbent deposits are positioned (e.g., confined) between the two sections of felted foam. For example, two sections of felted foam may be placed such that a first surface of the first section (on which the superabsorbent deposits are printed) faces the first surface (i.e. also on which the superabsorbent deposits are printed) of the second section. In other examples, superabsorbent deposits are only printed on one of the two sections of felted foam. The sections of felted foam can then be welded together along a border of the sections. In some embodiments, radio-frequency (RF) welding is used. That is, a radio-frequency electromagnetic field can be applied to cause heating of the felted foam in the desired region to create the welding effect. In alternative embodiments, an adhesive may be used to join the two sections of felted foam. The two sections of felted foam are thereby coupled together to confine the superabsorbent deposits and form the tea-bag structure.

At step 314, additional components of a dressing may be coupled to the felted foam (i.e., to the tea-bag structure) as desired for various dressings. For example, to manufacture the dressing shown in FIGS. 1-2, the drape 112 is coupled to the tea-bag structure 110 at step 314, for example using heat welding, RF welding, an adhesive, etc. In other embodiments various other dressing components such as drapes, films, absorbents, connection pads, tubes, sensors, removable liners, packaging, etc. are coupled to the felted foam at step 314. A dressing that includes a superabsorbent and felted foam tea-bag structure is thereby produced following process 300.

Although FIG. 3 presents the steps of process 300 in a particular order, the present disclosure contemplates various orders and overlaps between the steps of process 300. For example, in some embodiments, the felted foam is cut into multiple sections (at step 310) before the superabsorbent slurry is printed onto the felted foam material (at step 308). All such variations are within the scope of the present disclosure. In some embodiments, one or more steps of process 300 are omitted.

Process 300 can be adapted to produce various alternative embodiments of the dressing 104. For example, in some embodiments, a roll or strip that includes multiple tea-bag structures 110 can be produced. For example two strips of felted foam can be printed with superabsorbent deposits in groupings. The felted foam strips can be welded together along a border and between the groupings of deposits to form multiple-tea bag structures. The assembly can then be perforated or otherwise weakened between the groupings of deposits (i.e., along the welds between tea-bag structures) to facilitate separation of the tea-bag structures. The assembly can then be distributed and used in a roll or strip, from which a user can selectively remove the desired number of tea-bag structures for use in a bandage applied to a user.

As another example, in some embodiments a dressing may be formed having three or more layers of felted foam with superabsorbent deposits arranged amongst the layers. For example, a three-layer felted foam structure may be created in which superabsorbent is positioned between a first felted foam layer and a second felted foam layer, and between the second felted foam layer and a third felted foam layer, with the second felted foam layer of positioned between the first and third felted foam layers and welded to the first and third felted foam layers. Various such arrangements are possible.

As yet another example, in some embodiments a dressing may be formed having a single felted foam layer on which superabsorbent deposits are printed. In some embodiments of this example, the drape is sealed (e.g., RF welded) to the felted foam layer to confine the superabsorbent deposits between the felted foam layer and the drape. In some embodiments of this example, a film (e.g., a perforated silicone or polyurethane) is sealed to the felted foam layer to confine the superabsorbent deposits between the felted foam layer and the drape. Many such variations are possible.

Other arrangements and combinations of the elements described herein and shown in the Figures are also contemplated by the present disclosure. The construction and arrangement of the systems and apparatuses as shown in the various exemplary embodiments are illustrative only. Although only a few embodiments have been described in detail in this disclosure, many modifications are possible (e.g., variations in sizes, dimensions, structures, shapes and proportions of the various elements, values of parameters, mounting arrangements, use of materials, colors, orientations, etc.). For example, the position of elements can be reversed or otherwise varied and the nature or number of discrete elements or positions can be altered or varied. Accordingly, all such modifications are intended to be included within the scope of the present disclosure. Other substitutions, modifications, changes, and omissions can be made in the design, operating conditions and arrangement of the exemplary embodiments without departing from the scope of the present disclosure.

## Claims

1. A dressing, comprising:
a first foam layer having a pore density in a range between approximately 35.4 pores per cm (90 pores per inch) and approximately 141.7 pores per cm (360 pores per inch);
a superabsorbent material deposited on the first foam layer; and
a second foam layer having a pore density in a range between approximately 35.4 pores per cm (90 pores per inch) and approximately 141.7 pores per cm (360 pores per inch);
wherein the superabsorbent material is confined between the first foam layer and the second foam layer.

2. The dressing of Claim 1, comprising a drape coupled to the second foam layer, the drape comprising a hydrophilic polymer film.

3. The dressing of Claim 2, wherein the drape has a thickness in a range between approximately 15 microns and approximately 50 microns.

4. The dressing of Claim 2, wherein the drape comprises a silicone adhesive and an acrylic adhesive.

5. The dressing of Claim 2, comprising a connection pad coupled to the drape and configured to facilitate pneumatic communication between the first and second foam layers and a pump configured to draw a negative pressure at the wound dressing.

6. The dressing of Claim 1, wherein the first foam layer has a thickness in a range between approximately 2 millimeters and approximately 8 millimeters.

7. The dressing of Claim 1, wherein the superabsorbent material is deposited in a plurality of superabsorbent deposits arranged in a pattern on the first foam layer.

8. The dressing of Claim 7, wherein the pattern comprises an array such that the superabsorbent deposits are approximately evenly spaced.

9. The dressing of Claim 8, wherein the array has a spacing in a range between approximately 8 millimeters and approximately 15 millimeters.

10. The dressing of Claim 9, wherein the spacing is approximately 10 millimeters.

11. The dressing of Claim 1, wherein the first foam layer and the second foam layer are welded together.

12. A system, comprising:
a pump;
a tube coupled to the pump; and
a dressing according to any preceding claim coupled to the tube;
wherein the pump is operable remove air from the dressing when the dressing is applied to a wound.

13. A method of manufacturing a dressing, comprising:
obtaining an open-cell foam;
heating and compressing the open-cell foam to create a felted foam having a pore density in a range between approximately 35.4 pores per cm (90 pores per inch) and approximately 141.7 pores per cm (360 pores per inch);
printing a superabsorbent material onto a first surface of the felted foam;
dividing the felted foam into a first section and a second section; and
coupling the first section to the second section such that the first surface of the first section faces the first surface of the second section.

14. The method of Claim 13, wherein printing the superabsorbent material onto the first surface of the felted foam comprises:
dissolving the superabsorbent material in a solvent to form a slurry; and
depositing the slurry in a plurality of discrete deposits on the first surface of the felted foam, optionally wherein the slurry at least partially penetrates the felted foam,
optionally comprising allowing the solvent to evaporate, wherein the superabsorbent material becomes at least partially interlocked with the felted foam when the solvent evaporates.

15. The method of Claim 13, wherein heating and compressing the open-cell foam to create the felted foam comprises:
heating the open-cell foam to approximately 150 degrees Celsius; and
compressing the open-cell foam to approximately one-fifth of an original thickness of the open-cell foam.

## Patentansprüche

1. Ein Verband, aufweisend:
eine erste Schaumstoffschicht, die eine Porendichte in einem Bereich zwischen etwa 35,4 Poren pro cm (90 Poren pro Zoll) und etwa 141,7 Poren pro cm (360 Poren pro Zoll) aufweist;
ein superabsorbierendes Material, das auf der ersten Schaumstoffschicht aufgetragen ist; und
eine zweite Schaumstoffschicht, die eine Porendichte in einem Bereich zwischen etwa 35,4 Poren pro cm (90 Poren pro Zoll) und etwa 141,7 Poren pro cm (360 Poren pro Zoll) aufweist;
wobei das superabsorbierende Material zwischen der ersten Schaumstoffschicht und der zweiten Schaumstoffschicht eingeschlossen ist.

2. Der Verband nach Anspruch 1, aufweisend ein Abdeckmaterial, das mit der zweiten Schaumstoffschicht gekoppelt ist, das Abdeckmaterial aufweisend eine hydrophile Polymerfolie.

3. Der Verband nach Anspruch 2, wobei das Abdeckmaterial eine Dicke in einem Bereich zwischen etwa 15 Mikrometer und etwa 50 Mikrometer aufweist.

4. Der Verband nach Anspruch 2, wobei das Abdeckmaterial einen Silikonkleber und einen Acrylkleber aufweist.

5. Der Verband nach Anspruch 2, aufweisend ein Verbindungspad, das mit dem Abdeckmaterial gekoppelt und konfiguriert ist, um eine pneumatische Kommunikation zwischen der ersten und der zweiten Schaumstoffschicht und einer Pumpe zu ermöglichen, die konfiguriert ist, um einen Unterdruck an den Wundverband zu saugen.

6. Der Verband nach Anspruch 1, wobei die erste Schaumstoffschicht eine Dicke in einem Bereich zwischen etwa 2 Millimeter und etwa 8 Millimeter aufweist.

7. Der Verband nach Anspruch 1, wobei das superabsorbierende Material in einer Mehrzahl von superabsorbierenden Auflagen aufgetragen ist, die in einem Muster auf der ersten Schaumstoffschicht angeordnet sind.

8. Der Verband nach Anspruch 7, wobei das Muster ein Array aufweist, derart, dass die superabsorbierenden Auflagen ungefähr gleichmäßig beabstandet sind.

9. Der Verband nach Anspruch 8, wobei das Array eine Beabstandung in einem Bereich zwischen etwa 8 Millimeter bis etwa 15 Millimeter aufweist.

10. Der Verband nach Anspruch 9, wobei die Beabstandung etwa 10 Millimeter beträgt.

11. Der Verband nach Anspruch 1, wobei die erste Schaumstoffschicht und die zweite Schaumstoffschicht miteinander verschweißt sind.

12. Ein System, aufweisend:
eine Pumpe;
einen Schlauch, der mit der Pumpe gekoppelt ist; und
einen Verband nach einem der vorhergehenden Ansprüche, der mit dem Schlauch gekoppelt ist;
wobei die Pumpe betriebsfähig ist, um Luft aus dem Verband zu entfernen, wenn der Verband auf eine Wunde aufgebracht wird.

13. Ein Verfahren zum Herstellen eines Verbandes, aufweisend:
Erhalten eines offenzelligen Schaumsstoffs;
Erwärmen und Komprimieren des offenzelligen Schaumsstoffs, um einen gefilzten Schaumstoff zu erschaffen, der eine Porendichte in einem Bereich zwischen etwa 35,4 Poren pro cm (90 Poren pro Zoll) und etwa 141,7 Poren pro cm (360 Poren pro Zoll) aufweist;
Drucken eines superabsorbierenden Materials auf eine erste Oberfläche des gefilzten Schaumsstoffs;
Unterteilen des gefilzten Schaumsstoffs in einen ersten Abschnitt und einen zweiten Abschnitt; und
Koppeln des ersten Abschnitts mit dem zweiten Abschnitt derart, dass die erste Oberfläche des ersten Abschnitts der ersten Oberfläche des zweiten Abschnitts zugewandt ist.

14. Das Verfahren nach Anspruch 13, wobei das Drucken des superabsorbierenden Materials auf die erste Oberfläche des gefilzten Schaumstoffs aufweist:
Auflösen des superabsorbierenden Materials in einem Lösungsmittel, um eine Aufschlämmung auszubilden; und
Auftragen der Aufschlämmung in einer Mehrzahl von diskreten Auflagen auf der ersten Oberfläche des gefilzten Schaumsstoffs, optional wobei die Aufschlämmung den gefilzten Schaumsstoff mindestens teilweise durchdringt, optional aufweisend ein Erlauben des Verdampfens des Lösungsmittels, wobei das superabsorbierende Material mindestens teilweise mit dem gefilzten Schaumstoff ineinandergreift, wenn das Lösungsmittel verdampft.

15. Das Verfahren nach Anspruch 13, wobei das Erwärmen und Komprimieren des offenzelligen Schaumsstoffs zum Erschaffen des gefilzten Schaumssoffs aufweist:
Erwärmen des offenzelligen Schaumsstoffs auf etwa 150 Grad Celsius; und
Komprimieren des offenzelligen Schaumsstoffs auf etwa ein Fünftel einer ursprünglichen Dicke des offenzelligen Schaumsstoffs.

## Revendications

1. Pansement, comprenant :
une première couche de mousse ayant une densité de pores dans une plage entre approximativement 35,4 pores par cm (90 pores par pouce) et approximativement 141,7 pores par cm (360 pores par pouce) ;
un matériau superabsorbant déposé sur la première couche de mousse ; et
une seconde couche de mousse ayant une densité de pores dans une plage entre approximativement 35,4 pores par cm (90 pores par pouce) et approximativement 141,7 pores par cm (360 pores par pouce) ;
dans lequel le matériau superabsorbant est confiné entre la première couche de mousse et la seconde couche de mousse.

2. Pansement selon la revendication 1, comprenant un champ accouplé à la seconde couche de mousse, le champ comprenant un film polymère hydrophile.

3. Pansement selon la revendication 2, dans lequel le champ a une épaisseur dans une plage comprise entre approximativement 15 micromètres et approximativement 50 micromètres.

4. Pansement selon la revendication 2, dans lequel le champ comprend un adhésif de silicone et un adhésif acrylique.

5. Pansement selon la revendication 2, comprenant un tampon de liaison accouplé au champ et conçu pour faciliter une communication pneumatique entre les première et seconde couches de mousse et une pompe conçue pour aspirer une pression négative au niveau du pansement pour plaie.

6. Pansement selon la revendication 1, dans lequel la première couche de mousse a une épaisseur dans une plage comprise entre approximativement 2 millimètres et approximativement 8 millimètres.

7. Pansement selon la revendication 1, dans lequel le matériau superabsorbant est déposé en une pluralité de dépôts superabsorbants agencés selon un motif sur la première couche de mousse.

8. Pansement selon la revendication 7, dans lequel le motif comprend un réseau de telle sorte que les dépôts superabsorbants soient espacés approximativement uniformément.

9. Pansement selon la revendication 8, dans lequel le réseau a un espacement dans une plage comprise entre approximativement 8 millimètres et approximativement 15 millimètres.

10. Pansement selon la revendication 9, dans lequel l'espacement est d'approximativement 10 millimètres.

11. Pansement selon la revendication 1, dans lequel la première couche de mousse et la seconde couche de mousse sont soudées ensemble.

12. Système, comprenant :
une pompe ;
un tube accouplé à la pompe ; et
un pansement selon une quelconque revendication précédente accouplé au tube ;
dans lequel la pompe est utilisable pour éliminer l'air du pansement lorsque le pansement est appliqué sur une plaie.

13. Procédé de fabrication d'un pansement, comprenant :
l'obtention d'une mousse à cellules ouvertes ;
le chauffage et la compression de la mousse à cellules ouvertes pour créer une mousse feutrée ayant une densité de pores dans une plage entre approximativement 35,4 pores par cm (90 pores par pouce) et approximativement 141,7 pores par cm (360 pores par pouce) ;
l'impression d'un matériau superabsorbant sur une première surface de la mousse feutrée ;
la division de la mousse feutrée en une première section et une seconde section ; et
l'accouplement de la première section à la seconde section de telle sorte que la première surface de la première section soit face à la première surface de la seconde section.

14. Procédé selon la revendication 13, dans lequel l'impression du matériau superabsorbant sur la première surface de la mousse feutrée comprend :
la dissolution du matériau superabsorbant dans un solvant pour former une bouillie ; et
le dépôt de la bouillie en une pluralité de dépôts distincts sur la première surface de la mousse feutrée, éventuellement dans lequel la bouillie pénètre au moins partiellement la mousse feutrée, comprenant éventuellement le fait de laisser le solvant s'évaporer, dans lequel le matériau superabsorbant devient au moins partiellement enchevêtré avec la mousse feutrée lorsque le solvant s'évapore.

15. Procédé selon la revendication 13, dans lequel le chauffage et la compression de la mousse à cellules ouvertes pour créer la mousse feutrée comprennent :
le chauffage de la mousse à cellules ouvertes à approximativement 150 degrés Celsius ; et
la compression de la mousse à cellules ouvertes à approximativement un cinquième d'une épaisseur originale de la mousse à cellules ouvertes.
